# EUROPÄISCHE PATENTANMELDUNG

(11) **EP 0 528 314 A1**
(43) Veröffentlichungstag der Anmeldung: **24.02.1993**
(21) Anmeldenummer: 92113572.9
(22) Anmeldetag: 10.08.1992
(51) Int. Cl.: A61K 37/02, A61K 47/12, A61K 47/18, A61K 47/02

(54) **Verfahren zur Herstellung von Humanprotein-enthaltenden, gut verträglichen Arzneimitteln für Infusions- oder Injektionszwecke**

(30) Priorität: 15.08.1991 DE 4126984
(71) Anmelder: BOEHRINGER MANNHEIM GMBH, D-68298 Mannheim (DE)
(72) Erfinder: Woog, Heinrich, Dr., W-6947 Laudenbach (DE); Demmer, Fritz, Dr., W-6945 Hirschberg-Leutershausen (DE); Winter, Gerhard, Dr., W-6915 Dossenheim (DE); Markl, Hans-Jörg, W-6701 Ellerstadt (DE); Gruber, Werner, W-6943 Birkenau (DE)

(57) **Zusammenfassung**

Die vorliegende Erfindung betrifft ein Verfahren zur Herstellung von humanprotein-haltigen Arzneimitteln zur Anwendung als Injektions- oder Infusionslösung, wobei die Lösungen gut verträglich sind und einen pH-Wert von 2 - 7,4 und eine Pufferkapazität von bis zu 10 mVal/1 aufweisen.

## Beschreibung

Gegenstand der vorliegenden Erfindung ist ein Verfahren zur Herstellung von gut verträglichen Humanprotein-haltigen Arzneimitteln zur Anwendung als Infusions- oder Injektionslösung.

Humanproteine im Sinne der vorliegenden Erfindung sind körpereigene, nur in geringen Mengen vorkommende Proteine, die für therapeutische Zwecke eingesetzt werden, wie z.B. t-PA (tissue plasminogen activator), G-CSF (granulocyte colony stimulating factor), Streptokinase, Urokinase, Interferon oder EPO (Erythropoietin), bzw. deren rekombinant hergestellten Derivate, die im wesentlichen ähnliche oder vergleichbare pharmakologische Eigenschaften besitzen.

In der Europäischen Patentanmeldung EP 0,430,200 werden Humanprotein-haltige Arzneimittel zur subkutanen oder intramuskulären Applikation beschrieben, die durch Zusatz von Aminosäuren eine bessere Bioverfügbarkeit und eine bessere Verträglichkeit im Vergleich zu bekannten Darreichungsformen aufweisen.

Stabilisierte Humanprotein-haltige Arzneimittel, die u.a. Harnstoff und verschiedene Aminosäuren enthalten, sind aus EP 0,306,824 bekannt, wobei dort als Humanproteine insbesondere EPO und G-CSF beispielhaft erwähnt sind.

Ferner werden in EP 0,456,153 galenische wäßrige Formulierungen von EPO zur Herstellung von Injektionspräparaten zur subkutanen oder intramuskulären Applikation beschrieben, die einen pH-Wert von 6 - 8 aufweisen und zur Stabilisierung ausschließlich ein Alkalimetallphosphat oder Alkalimetallhalogenid enthalten.

Die gentechnologische Herstellung der oben genannten Humanproteine ist beispielsweise aus den folgenden Patentanmeldungen bekannt: In den PCT-Anmeldungen WO 85/02610 und WO 86/03520 sind Verfahren zur gentechnologischen Erzeugung von rh-EPO (recombinantes humanes Erythropoietin) beschrieben. Ferner ist die Herstellung von Polypeptiden mit erythropoietin-ähnlicher Wirkung beschrieben in EP 0,409,113; EP 0,357,804; WO 86/02100 und WO 91/05867. Aus dem stand der Technik sind weiterhin Verfahren zur Herstellung von anderen rekombinanten Proteinen bekannte, beispielsweise von Polypeptiden mit plasminogenaktivator-ähnlicher Wirkung aus WO 90/09437; EP 0,227,462; EP 0,400,545 oder EP 0,440,763. Die Herstellung von Polypeptiden mit G-CSF ähnlicher Wirkung ist beispielsweise bekannt aus EP 91 107 429.2 oder PCT/EP91/00192.

Allgemein bezieht sich der Begriff "rekombinant" auf solche Humanproteine, die mit Hilfe der rekombinaten DNA-Technologie hergestellt werden. Diese Verfahren umfassen die Klonierung des Gens, das für das jeweilige Humanprotein kodiert, die Insertion der entsprechenden cDNA oder genomischen DNA in einen geeigneten Vektor, wie z.B. in bakterielle Plasmide, und die Transformation dieser rekombinanten Plasmide in geeignete Wirtszellen. Das klonierte Gen wird dann in der Wirtszelle exprimiert und das entsprechende Humanprotein wird auf an sich bekannte Art und Weise isoliert.

Es wurde allerdings festgestellt, daß Humanprotein-haltige Injektions- oder Infusionslösungen verschiedener Hersteller aufgrund unterschiedlicher Zusammensetzungen in der galenischen Formulierung oder aufgrund geringer struktureller Unterschiede der Wirkstoffe hinsichtlich der Aminosäuresequenz oder des Glykosylierungsmusters des Proteins unterschiedlich verträglich waren. Obwohl die aus dem Stand der Technik bekannten Lösungen im wesentlichen isotonische Lösungen waren, die an sich ohne größere Probleme gut verträglich sein sollten, wurden bei der Applikation unangenehme Nebenwirkungen festgestellt. Bei der Verabreichung von beispielsweise EPO-haltigen Injektionslösungen hatten Patienten oft über Schmerzen an der Einstichstelle geklagt, die während und nach der Applikation auftraten. In Abhängigkeit von der jeweils verwendeten galenischen Formulierung traten bei vielen Patienten häufig brennende Schmerzen auf, wenn insbesondere solche Injektionslösungen verabreicht wurden, die zur Stabilisierung humanes Serumalbumin und Zitratpuffer als Zusatz enthielten. In einigen Fällen kam es bei den Patienten zu erhöhter Temperatur, hohem Blutdruck, Urtikaria, Rückenschmerzen, Nausea oder auch zum Schock.

Ferner hat sich gezeigt, daß Injektionslösungen mit einem relativ geringen Gehalt an Wirkstoff nicht hinreichend gut stabilisert werden können. So waren beispielsweise Arzneimittelformulierungen, die als Humanprotein EPO in einer Menge von z. B. 500 - 20.000 U enthielten, nicht ausreichend stabil. So konnte nachgewiesen werden, daß einige galenische Formulierungen die unerwünschte Bildung von Aggregaten oder Agglomeraten der Humanproteine, insbesondere bei längerer Lagerung begünstigen. Dadurch kann es bei der Anwendung derartiger Präparate zu immunologischen Problemen kommen.

Es stellte sich daher die Aufgabe, verbesserte Injektionslösungen bereitzustellen, die gut verträglich sind. Außerdem sollten diese Lösungen über längere Zeit weitgehend aggregatfrei gelagert werden können. Sie sollten reproduzierbar gut verträglich appliziert werden können, und eine möglichst schmerzfreie Applikation gewährleisten.

Die erfindungsgemäße Aufgabe wird dadurch gelöst, daß man humanproteinhaltige Arzneimittel zur Anwendung als Infusions- oder Injektionslösungen herstellt, deren pH-Wert im sauren oder neutralen Bereich liegt und die eine möglichst geringe Pufferkapazität besitzen. Die Pufferkapazität liegt insbesondere im Bereich von 0 - 10 mVal/1, bevorzugt 0 - 6 mVal/1. Bevorzugt beträgt die Pufferkapazität bis zu 3 mVal/1, 2 mVal/1 oder 1 mVal/1, insbesondere bis zu 0,5 mVal/1. Der pH-Wert liegt im Bereich von etwa 2 - 7,4, je nach Stabilität des verwendeten Humanproteins in saurem Medium. Insbesondere kommt ein pH-Bereich von etwa 3,8 - 7,4 in Frage, bevorzugt 4,5 - 7,4.

Infusionslösungen, die für die intravenöse Gabe geeignet sind, besitzen vorzugsweise eine Pufferkapazität von bis 3 mVal/1 oder bis zu 1 mVal/1, bevorzugt bis zu 0,3 mVal/1 oder bis zu 0,1 mVal/1, insbesondere bis zu 0,05 mVal/1. Injektionslösungen, die für die subkutane Gabe geeignet sind, besitzen vorzugsweise eine Pufferkapazität von bis zu 1 mVal/1, 0,2 mVal/1 oder bis zu 0,1 mVal/1, insbesondere von bis zu 0,05 mVal/1.

Der obere pH-Wert der applizierbaren Arzneimittellösung darf nicht wesentlich über dem Neutralpunkt liegen (der pH-Wert des Blutes liegt zwischen 7,2 und 7,4), weil Humanproteine im alkalischen Bereich nicht beständig sind. Die intravenöse und die subkutane Applikation unterscheiden sich deshalb, weil durch das intravenöse Anfluten von Blut und die im Blut enthaltenen Puffer eine schnellere Angleichung an physiologische pH-Verhältnisse möglich ist, als dies subkutan erfolgen kann.

Die Pufferkapazität wird allgemein definiert als diejenige äquivalente Menge (Val) an Säure oder Lauge, die erforderlich ist, um den pH-Wert einer Lösung mit dem Volumen von einem Liter um eine pH-Einheit zu verändern. Falls einbasige Säuren oder Basen für die Titration verwendet werden, entspricht die Angabe Val/1 der verwendeten Säure oder Base der molaren Menge Mol/l dieser Säure. Da im vorliegenden Fall die verwendeten Lösungen einen pH-Wert im sauren Bereich aufweisen, kann alternativ die Pufferkapazität auch als diejenige Menge einer beispielsweise 0,1 n NaOH-Lösung definiert werden, die benötigt wird, um den pH-Wert einer Lösung von einem Liter um eine pH-Einheit anzuheben. Die Humanprotein-haltigen Arzneimittellösungen enthalten bei der Bestimmung der Pufferkapazität die üblichen pharmazeutischen Hilfs- oder Trägerstoffe.

Die Bestimmung der Pufferkapazität der Humanprotein-haltigen Arzneimitteln erfolgt ausgehend von den fertig applizierbaren Injektions- oder Infusionslösungen, die neben dem Wirkstoff die sonst in der pharmazeutischen Praxis üblichen Hilfs- oder Zusatzstoffen enthalten. In der Regel besitzen die Lösungen einen sauren pH-Wert zur Stabilisierung des Proteins. Durch Titration mit Basen wird die entsprechende Menge der Base bestimmt, die erforderlich ist, um den pH-Wert der Lösung um eine Einheit zu erhöhen.

Bevorzugte Grenzen für die Pufferkapazität in der Infusions- oder Injektionslösung sind für die intravenöse Gabe bis zu 2,4 ml einer 0,1 n Natronlauge, bevorzugt bis zu 0,5 ml. Dies entspricht einer Menge an Lauge von 0,24 mMol bzw. 0,05 mMol. Für die subkutane Gabe werden bevorzugt bis zu 1 ml einer 0,1 n NaOH-Lösung, insbesondere bis zu 0,2 ml einer 0,1 n NaOH-Lösung verwendet. Dies entspricht einer Menge an Lauge von bis zu 0,1 mMol, bzw. bis zu 0,02 mMol.

Ferner hat sich gezeigt, daß es vorteilhaft ist, wenn die fertig applizierbaren Injektions- oder Infusionslösungen eine möglichst geringe Titrationsacidität von bis zu 10 mVal/1 aufweisen, insbesondere bis zu 5 mVal/1.

Bevorzugte Grenzen für die Titrationsacidität der Infusions- oder Injektionslösungen sind für die intravenöse Applikation bis zu 10 ml, bevorzugt bis zu 5 ml, 3 ml oder 1 ml einer 0,1 n NaOH-Lösung. Dies entspricht einer Titrationsacidität von bis zu 1 mmol/l, bzw. bis zu 0,3 mmol/l oder 0,1 mmol/l. Für die subkutane Applikation werden bevorzugt bis zu 5 ml, insbesondere bis zu 2 ml oder bis zu 0,5 ml einer 0,1 n NaOH-Lösung verwendet. Die Titrationsacidität beträgt in diesem Fall bis zu 0,5 mmol/l, bzw. bis zu 0,2 mmol/l oder 0,05 mmol/l.

Die Titrationsacidität bzw. -basizität wird allgemein definiert als diejenige Menge an Lauge bzw. Säure, die erforderlich ist, um den pH-Wert einer Lösung mit dem Volumen von einem Liter auf den pH-Wert des Blutes (etwa 7,2 - 7,4) einzustellen. Im vorliegenden Fall kann die Titationsacidität alternativ auch als diejenige Menge einer beispielsweise 0,1 n NaOH-Lösung definiert werden, die erforderlich ist, um den pH-Wert von einem Liter einer Lösung auf den des Blutes (etwa 7.3) anzuheben. Die Humanprotein-haltigen Arzneimittellösungen enthalten bei der Bestimmung der Pufferkapazität die üblichen pharmazeutischen Hilfs- oder Zusatzstoffe. Das Verfahren zur BeStimmung der Titrationsacidität erfolgt in analoger Weise wie die Bestimmung der Pufferkapazität, indem man von der fertig applizierbaren Injektions-oder Infusionslösung ausgeht und diejenige Menge an Base bestimmt, die erforderlich ist, um den pH-Wert von etwa 7 der Lösung einzustellen.

Der pH-Bereich für eine weitgehend schmerzfrei applizierbare Infusions- oder Injektionslösung mit relativ niedriger Pufferkapazität und Titrationsacidität liegt in Abhängigkeit des jeweils verwendeten Humanproteins im Bereich von etwa 2 - 7,4, wobei als untere Grenze insbesondere die pH-Werte 3,8 - 6,0, bevorzugt 4,5 - 7,0 bzw. 5,5 - 6,0 in Frage kommen. Als obere Grenze des pH-Bereiches werden vorzugsweise pH-Werte der Lösungen verwendet, die in der Nähe des pH-Wertes des Blutes liegen. Für intravenöse Applikationen werden vorzugsweise Lösungen mit einem pH-Wert von 6 - 7,4, insbesondere 6,8 - 7,2 verwendet. Für subkutane Applikationen werden vorzugsweise Lösungen mit einem pH-Wert von 6,5 - 7,2, insbesondere 7,0 - 7,2 verwendet.

Außer der natürlich vorkommenden Form der Humanproteine können auch geeignete Muteine verwendet werden. Unter dem Begriff "Muteine" werden in der Regel solche Humanproteine verstanden, deren Aminosäuresequenz sich durch mindestens eine Aminosäure von der natürlichen Sequenz unterscheidet. Diese Unterschiede können darin bestehen, daß eine oder mehrere, vorzugsweise 1-10 Aminosäuren in der natürlichen Sequenz durch andere Aminosäuren ausgetauscht sind, oder daß eine oder mehrere Aminosäuren an das N- oder C-terminale Ende hinzugefügt bzw. auch weggelassen werden. Man spricht dann von N- oder C-terminalen Verlängerungen bzw. N- oder C-terminalen Deletionen. Die zuvor genannten Möglichkeiten lassen sich gegebenenfalls auch miteinander kombinieren, d.h. das N-terminale Ende der natürlichen Sequenz kann beispielsweise unter gleichzeitiger Verkürzung des C-terminalen Endes verlängert werden, wobei gegebenenfalls gleichzeitig auch eine oder mehrere Aminosäuren durch andere Aminosäuren ausgetauscht werden können. Im Hinblick auf die jeweilige Indikationsrichtung sollten die so erhaltenen Fragmente im wesentlichen die gleichen grundlegenden therapeutischen Eigenschaften und Wirkungen aufweisen wie die natürlichen Humanproteine.

Für die Verträglichkeit spielt auch eine Rolle, ob eine Darreichungsform konserviert werden muß. Alle Konservierungsmittel weisen eine mehr oder weniger große Allergierate auf. Um Keimfreiheit zu garantieren, ist ihr Einsatz aber nicht immer zu vermeiden. Nach den der Anmeldung zugrunde liegenden Untersuchungen ist es möglich, die Konservierungsmittel so bei der Herstellung der Injektionslösungen einzusetzen, daß sowohl eine weitestgehende Keimfreiheit garantiert ist, als auch Nebenwirkungen der Konservierungsmittel fast vollständig ausgeschlossen werden. Dies läßt sich dadurch erreichen, daß entweder das Konservierungsmittel noch während der Herstellung entfernt wird, oder - wenn der Verbleib des Konservierungsmittels in der Lösung unvermeidlich ist - das Konservierungsmittel im Hinblick auf eine möglichst geringe Allergierate ausgewählt wird und/oder seine erforderliche Konzentration in der Lösung minimiert wird, indem seine Absorption an Materialien ausgeschlossen wird, mit denen die Lösung in Berührung kommt.

Die flüssigen oder auch lyophilisierten Arzneimittel enthalten gegebenenfalls üblich pharmazeutische Hilfsstoffe, wie z.B. Stabilisierungsmittel oder organische hydrophile Polymere. Als Stabilisierungsmittel sind beispielsweise geeignet Oligosaccharide, wie z.B. Sucrose, Tetralose, Lactose, Dextrane mit einem Molekulargewicht von etwa 10,000 - 2,000,000. Organische hydrophile Polymere sind Makromoleküle mit einem Kohlenstoffgrundgerüst, das aus hydrophilen monomeren Einheiten mit gegebenenfalls polaren Seitengruppen aufgebaut ist, wie beispielsweise Polyethylenglykol oder Polyvinylpyrrolidon.

Ferner enthalten die Arzneimittelzubereitungen pharmazeutisch übliche Puffer, wie z.B. Alkaliphosphate, Salze von organischen oder anorganischen Säuren oder Aminosäuren. Die Zusammensetzung der verschiedenen Puffersubstanzen in der Rezeptur wird so gewählt, daß eine möglichst geringe Pufferkapazität der fertig applizierbaren Injektions- oder Infusionslösung resultiert. Dies kann dadurch geschehen, indem man eine möglichst geringe Menge der Puffersubstanzen einsetzt, wobei insbesondere die Gesamtmenge des Puffers eine Konzentration in der Arzneimittellösung von 100 mmol/l nicht übersteigen sollte. Bevorzugt werden Puffersubstanzen in einer Konzentration von 10 - 100 mmol/l, insbesondere von 20 - 60 mmol/l eingesetzt. Alternativ dazu ist es auch möglich, die einzelnen Puffersubstanzen so auszuwählen, daß sie sich in ihrer im wesentlichen im sauren oder basischen Pufferbereich liegenden Wirkung gegenseitig kompensieren. In diesem Fall kann die Gesamtmenge an Puffersubstanzen bis zu 200 mmol/l in der fertig applizierbaren Arzneiform betragen.

Die lyophilisierten Arzneimittel enthalten vorzugsweise zusätzlich einen Gerüstbildner, der beim Einfrieren der wässrigen Lösung eine kristalline Matrix ausbildet, die auch während des sich anschließenden Lyophilisierens und bei der längeren Lagerung des Lyophilisats unter verschiedenen äußeren Bedingungen in ihrer Struktur stabil bleibt. In diesem Sinne kommen als geeignete Gerüstbildner Mannitol oder Glycin in Frage.

Die so hergestellten Arzneimittel kommen vorzugsweise in Form von Lyophilisaten in den Handel. Sie können als Single-Dose Präparate verwendet werden, wobei eine bestimmte Menge des Humanproteins in einer Injektionsflasche oder Karpule vorliegt und das Lyophilisat durch Zugabe der entsprechenden Menge an Rekonstitutionslösung gelöst wird.

Die Rekonstitutionslösung kann bereits die erforderlichen Menge an Lauge enthalten, die nötig ist, um den gewünschten pH-Wert der injektionsfertigen Lösung einzustellen. Daneben können die üblichen isotonischen Zusätze verwendet werden.

Die rekonstituierte Lösung wird dann auf eine Injektionsspritze aufgezogen und kann direkt dem Patienten appliziert werden. Sogenannte Single-Dose Präparate enthalten beispielsweise rh-EPO in einer Menge von 2.000 bis 20.000, bevorzugt 5000, 10.000 oder 15.000 U. Bei entsprechend größerem Einsatz des Humanproteins können auch Multi-Dose-Präparate mit beispielsweise bis zu 200.000 U hergestellt werden. In diesem Fall wird ein größeres Volumen (etwa 5 - 10 ml) als Rekonstitutionslösung verwendet, wobei diese Lösung dann für mehrere Applikationen verwendbar ist. Die zu applizierende Menge des Humanproteins kann in diesem Fall individuell vom Arzt festgelegt werden, bzw. für mehrere Applikationen bei verschiedenen Patienten eingesetzt werden.

Die spezifische Aktivität des zur Herstellung der Injektions- oder Infusionslösungen eingesetzten EPO beträgt vorzugsweise etwa 160.000 IU pro Absorptionseinheit bei 280 nm (vgl. EP 0,209,539).

Im folgenden wird die Erfindung anhand von Beispielen im einzelnen beschrieben. Als Wirkstoff wird stellvertretend für die Klasse der Humanproteine jeweils rh-EPO oder G-CSF eingesetzt. In gleicher Weise können aber auch andere Humanproteine verwendet werden.

Zur Herstellung der bei der Erfindung einsetzbaren Injektionslösungen werden in einem sterilen, mit Rührwerk versehenen V2A-Doppelmantelkessel die Hilfsstoffe in Wasser gelöst. Die wesentlichen Hilfsstoffe sind pharmazeutisch übliche Puffer, Komplexbildner, Stabilisatoren und Netzmittel. Für die Einstellung des physiologisch optimalen Bereichs für die intravenöse und die subkutane Anwendung sind als Puffer insbesondere geeignet: Glykokoll, Natriumcitrat, primäres Kaliumphosphat, sekundäres Natriumphosphat, Carbonat und verträgliche Salze von Aminosäuren, wie z. B. Arginin, Lysin, Ornithin, Glycin oder Histidin, außerdem die Natrium- und Kaliumsalze der Apfelsäure, Maleinsäure, Fumarsäure, Weinsäure und Asparaginsäure und Kombinationen dieser Substanzen. Die Puffer werden in einer Konzentration von etwa 1 bis etwa 100 mmol/l Lösung eingesetzt. Der Wirkstoff wird zu der Lösung hinzugegeben und es wird zum Endvolumen aufgefüllt und durchgerührt. Die Ansatzlösung wird über einen Membranfilter mit 0,2m Porenweite sterilfiltriert. Die dabei gewonnene Lösung wird zu 0,5 ml in Injektionsflaschen unter aseptischen Bedingungen abgefüllt und danach in einer Lyophilisationsanlage getrocknet.

Die oben beschriebenen Rezepturen sind auch als spritzfertige Lösungen stabil. Bei ihrer Herstellung wird die erhaltene Lösung nicht lyophilisiert, sondern nach der Sterilfiltration direkt in eine Ampulle oder Injektionsflasche zu je 1 ml pro Behältnis abgefüllt.

Die Anwesenheit von Konservierungsmitteln kann die Verträglichkeit von Humanprotein-Lösungen beeinträchtigen. Hinzu kommt, daß Konservierungsmittel mehr oder weniger stark mit Humanproteinen reagieren und diese dabei inaktivierten. Trotzdem kann auf die bakterizid wirkenden Konservierungsmittel nicht immer verzichtet werden, weil Keime beispielsweise beim Abfüllen in die Lösung eindringen können, oder weil, wenn aus einem sterilen Lyophilisat in einem Multidose-Behältnis eine Injektionslösung bereitet wird, diese bis zum vollständigen Verbrauch konserviert werden muß.

Unproblematisch ist der Konservierungsmittelzusatz bei der Herstellung des Präparats, wenn das Konservierungsmittel so ausgewählt wird, daß es bei der Lyophilisation verdampft oder wegsublimiert. Konservierungsmittel, die eine entsprechende Flüchtigkeit aufweisen, sind z. B. Chloreton, Benzylalkohol. Die Verträglichkeit konservierter Injektionslösungen läßt sich verbessern, indem die Konservierungsmittel möglichst gering dosiert werden (bis zu 2 %). Die Verträglichkeit läßt sich darüber hinaus auch durch eine günstige Auswahl der Konservierungsmittel verbessern. Konservierungsmittel, die eine geringe Allergierate aufweisen - und im übrigen auch mit dem Humanprotein relativ langsam reagieren -, sind z. B. Chlorbutanol, Benzylalkohol und Benzalkoniumchlorid.

Zur Herstellung der pharmazeutischen Darreichungsformen, die die Humanproteine enthalten, werden die üblichen pharmazeutischen Hilfs- oder Zusatzsoffe verwendet. Ferner können Stabilisierungs- oder Solubilisierungsmittel, wie z.B. die basischen Aminosäuren Arginin, Lysin oder Ornithin, zugesetzt werden. Ferner können als Aminosäure verwendet werden: Aminoessigsäure, Glutaminsäure, Isoleucin, Leucin, Phenylalanin, Threonin, sowie weitere in den Patentanmeldungen EP 0 430 200 oder EP 0 306 824 genannte Aminosäuren. Die Darreichungsform kann als Lyophilisat oder auch als gebrauchsfertige Infusions- oder Injektionslösung in den Handel gebracht werden.

Wird ein Lyophilisat gewählt, so kann die pharmazeutische Verpackungseinheit zusätzlich die zur Rekonstitution erforderlichen Lösungsmittel enthalten. Diese sind in der Regel so auf das entsprechende Lyophilisat abgestellt, daß bei der Mischung die spritzfertigen Lösungen mit den erfindungsgemäßen Eigenschaften erhalten werden. Das Lyophilisat kann die zur Einstellung des vorteilhaften PH-Bereiches erforderlichen Mengen an basischen Mitteln bereits ganz oder teilweise enthalten, so daß die Rekonstitution im wesentlichen mit destilliertem Wasser für Injektionszwecke erfolgt. Andererseits ist es auch prinzipiell möglich, daß die Rekonstitutionslösung die erforderliche Menge an basischen Mitteln enthält, um den pH-Bereich der spritzfertigen Lösung einzustellen. Ferner können sowohl Lyophilisat als auch die Rekonstitutionslösung Mittel enthalten, die die Herstellung einer isotonischen Lösung gewährleisten.

Bei der Herstellung der pharmazeutischen Verpackungseinheit werden die Darreichungsformen in der Regel mit einem Beipackzettel versehen, auf dem unter anderem der Hinweis enthalten ist, daß die Infusions- oder Injektionslösungen eine gut verträgliche, schmerzfreie Applikation ermöglichen.

Im folgenden werden humanproteinhaltige Rezepturen beschrieben, die aufgrund ihres günstigen pH-Wertes, bzw. ihrer günstigen Pufferkapazität und ihrer günstigen Titrationsacidität, völlig schmerzfrei sowohl intravenös als auch subkutan appliziert werden können. Die Herstellung der in den Beispielen genannten Lösungen erfolgte wie oben angegeben:

### Beispiel 1

| Erythropoietin | 5.000 U | 5.000 U | 5.000 U |
|---|---|---|---|
| Harnstoff | 25,000 mg | 25,000 mg | 25,000 mg |
| Calciumchlorid | 0,151 mg | 0,151 mg | 0,151 mg |
| NaCl | 2,500 mg | 2,500 mg | 2,500 mg |
| Polysorbat 20 | 0,250 mg | 0,250 mg | 0,250 mg |
| Natriumdihydrogenphosphat | - | 1,190 mg | - |
| Natriumhydrogenphosphat | - | 9.965 mg | - |
| Citronensäuremonohydrat | 10,500 mg | - | q.s. bis |
| Natronlauge | 5,890 mg | - | pH 6,8 |
| Weinsäure | - | - | 10,200 mg |
| Aminoessigsäure | 37,500 mg | 37,500 mg | 37,500 mg |
| L-Leucin | 5,000 mg | 5,000 mg | 5,000 mg |
| L-Isoleucin | 5,000 mg | 5,000 mg | 5,000 mg |
| L-Threonin | 1,250 mg | 1,250 mg | 1,250 mg |
| L-Glutaminsäure | 1,250 mg | 1,250 mg | 1,250 mg |
| L-Phenylalanin | 2,500 mg | 2,500 mg | 2,500 mg |
| Wasser | 2,14 - | 2,14 - | 2,14 - |
| | 5,35 ml | 5,35 ml | 5,35 ml |
| pH-Wert der in 1 ml Wasser für Injektionszwecke gelösten Lyophilisatform | 6,6 | 7,2 | 6,8 |
| Pufferkapazität: 0,01 mmol/l (0,1 ml 0,1 n NaOH) Titrationsacidität: 0,015 mmol/l (0,15 ml 0,1 n NaOH) | | | |

Die in dem Beispiel beschriebenen Rezepturen sind sowohl als Lyophilisate als auch als spritzfertige Lösungen lagerstabil. Die spezifische Aktivität des verwendeten EPO beträgt ca. 160.000 IU pro Absorptionseinheit bei 280 nm.

### Beispiel 2 (EPO-Lyophilisat zu 2.000 U/vial)

| | | | |
|---|---|---|---|
| EPO | 2.000 U | 2.000 U | 2.000 U |
| Harnstoff | 5,00 mg | 0,50 mg | 0 |
| Polysorbat | 0,10 mg | 0,10 mg | 0,10 mg |
| NaCl | 0,60 mg | 0,60 mg | 0,60 mg |
| Weinsäure | 4,08 mg | 4,08 mg | 4,08 mg |
| Natronlauge | 9,50 mg | 9,50 mg | 9,50 mg |
| CaCl₂ 2H₂O | 0,08 mg | - | - |
| Aminoessigsäure | 15,00 mg | 15,00 mg | 15,00 mg |
| L-Glutaminsäure | 0,50 mg | 0,50 mg | 0,50 mg |
| L-Isoleucin | 2,00 mg | 2,00 mg | 2,00 mg |
| L-Leucin | 2,00 mg | 2,00 mg | 2,00 mg |
| L-Phenylalanin | 1,00 mg | 1,00 mg | 1,10 mg |
| L-Threonin | 0,50 mg | 0,50 mg | 0,50 mg |
| Wasser für Injektionszwecke | 2,14-5,35 ml | 2,14-5,35 ml | 2,14-5,35 ml |
| pH-Wert der rekonst. Lösungen | 7,0 | 7,0 | 7,0 |
| Pufferkapazität : 0,01 mmol/l (0,1 ml 0,1 n NaOH) Titrationsacidität: 0,015 mmol/l (0,15 ml 0,1 n NaOH) | | | |

Die in dem Beispiel beschriebenen Rezepturen sind sowohl als Lyophilisate als auch als spritzfertige Lösungen lagerstabil.

### Beispiel 3

### Bildung von Oligomeren

rh-EPO enthaltende Arzneimittel wurden hinsichtlich ihrer Tendenz zur Ausbildung von Oligomeren untersucht. Die aus dem Stand der Technik bekannten Formulierungen wurden hierbei mit dem erfindungsgemäßen Formulierungen verglichen. Die Arzneimittel wurden als Lyophilisate bei verschiedenen Temperaturen über einen längeren Zeitraum gelagert und anschließend mit destilliertem Wasser rekonstituiert. Der prozentuale Anteil an Oligomeren in den Formulierungen wurde durch Western-Blotting bestimmt. Bei Formulierungen, die humanes Serumalbumin und Zitrat enthielten, wurden je nach Hersteller Aggregate mit einem Anteil von 16 %, 8 % und 3 % gefunden. Die nach dem erfindungsgemäßen Verfahren hergestellten Lösungen waren dagegen praktisch aggregatfrei.

### Beispiel 4

| G-CSF-Rezepturen mit pH-Wert 4,5 | |
|---|---|
| rhG-CSF | 0,175 mg |
| Natriumchlorid | 1,500 mg |
| Polysorbat 80 | 0,050 mg |
| Aminoessigsäure z.A. | 6,550 mg |
| L-Leucin | 0,500 mg |
| L-Isoleucin | 0,500 mg |
| L-Threonin | 0,125 mg |
| L-Glutaminsäure | 0,125 mg |
| L-Phenylalanin | 0,250 mg |
| NaOH 0,1 molar ad pH 4,5 | 0,000 mg |
| Wasser für Injektionszwecke | +492,225 mg |
| pH-Wert der in 0,5 ml Wasser für Injektionszwecke rekonstituierten Lösung: 4,5 Pufferkapazität : 3,0 mmol/l NaOH (30 ml 0,1 n NaOH) Titrationsacidität: 5,0 mmol/l NaOH (50 ml 0,1 n NaOH) | |

Die Bezeichnung rhG-CSF steht für recombinant human G-CSF.

### Beispiel 5

| G-CSF-Rezeptur mit pH-Wert 3,8 - 4,0 | |
|---|---|
| rhG-CSF | 0,175 mg |
| Natriumchlorid | 1,500 mg |
| Polysorbat 80 | 0,050 mg |
| Aminoessigsäure z.A. | 5,750 mg |
| L-Leucin | 0,500 mg |
| L-Isoleucin | 0,500 mg |
| L-Threonin | 0,125 mg |
| L-Glutaminsäure | 0,125 mg |
| L-Phenylalanin | 0,250 mg |
| HCl 0,1 molar ad pH 3,8 - 4,0 | 0,000 mg |
| Wasser für Injektionszwecke | +493,025 mg |
| pH-Wert der in 0,5 ml Wasser für Injektonszwecke rekonstituierten Lösung: 3,9 Pufferkapazität : 5,8 mmol/l NaOH (58 ml 0,1 n NaOH) Titrationsacidität: 10 mmol/l NaOH (100 ml 0,1 n NaOH) | |

### Beispiel 6

| G-CSF-Rezepturen mit pH 4 | | | |
|---|---|---|---|
| rhG-CSF | 0,175 mg | 0,175 mg | 0,175 mg |
| Harnstoff | 2,500 mg | 0,250 mg | 0,000 mg |
| Natriumchlorid | 1,500 mg | 1,500 mg | 1,500 mg |
| Polysorbat 80 | 0,050 mg | 0,050 mg | 0,050 mg |
| Aminoessigsäure z.A. | 3,750 mg | 5,550 mg | 5,750 mg |
| L-Leucin | 0,500 mg | 0,500 mg | 0,500 mg |
| L-Isoleucin | 0,500 mg | 0,500 mg | 0,500 mg |
| L-Threonin | 0,125 mg | 0,125 mg | 0,125 mg |
| L-Glutaminsäure | 0,125 mg | 0,125 mg | 0,125 mg |
| L-Phenylalanin | 0,250 mg | 0,250 mg | 0,250 mg |
| Wasser für Injektionszwecke | +492.525 mg | +492.975 mg | +493.025 mg |
| pH-Wert der in 0,5 ml Wasser f. Injektionszwecke gelösten Lyophilisatform | 4,0 | 4,0 | 4,0 |
| Pufferkapazität : 5,8 mmol/l NaOH (58 ml 0,1 n NaOH) Titrationsacidität: 10 mmol/l NaOH (100 ml 0,1 n NaOH) | | | |

Die in dem Beispiel beschriebenen Rezepturen sind sowohl als Lyophilisat als auch als spritzfertige Lösungen lagerstabil.

### Beispiel 7

Vergleich mit den aus dem Stand der Technik bekannten Injektions- oder Infusionslösungen

Es wurden Lösungen nach den in den Patentanmeldungen EP 0 430 200 und EP 0 456 154 genannten Methoden hergestellt, wobei als Humanprotein jeweils EPO verwendet wurde.
Lösung 1: gemäß EP 0 430 200
0,2 m Arginin, 0,2 m Lysin
eingestellt auf pH 4,5
Lösung 2: gemäß EP 0 430 200
0,2 m Arginin; 0,2 m Lysin
eingestellt auf pH 7,5
Lösung 3: gemäß EP 0 456 153
16,76 g Na₂HPO₄
0,5 g NaH₂PO₄
5,845 g NaCl
Wasser für Injektionszwecke ad 1,0 l
pH-Wert 7,8

**Tabelle**

| Pufferkapazität und Titrationsacidität bzw. -basizität der Lösungen 1-3: | | | |
|---|---|---|---|
| | Lösung 1 | Lösung 2 | Lösung 3 |
| Pufferkapazität | 11,25 mmol/l | 116,1 mmol/l | 19 mmol/l |
| | NaOH von pH | HCl von pH | HCl von pH |
| | 4,5 auf 5,5 | 7,5 auf 6,5 | 7,8 auf 6,8 |
| Titrationsacidität bzw. -basizität | 46,65 mmol/l | 6,9 mmol/l | 16,6 mmol/l |
| | NaOH von pH | HCl von pH | HCl von pH |
| | 4,5 auf 7,4 | 7,5 auf 7,4 | 7,8 auf 7,4 |

## Patentansprüche

1. Verfahren zur Herstellung von gut verträglichen Humanprotein-haltigen Arzneimitteln zur Anwendung als Injektions- oder Infusionslösung, dadurch gekennzeichnet, daß man eine Humanprotein-haltige Lösung zusammen mit verträglichen pharmazeutischen Hilfsstoffen zu Arzneimittelformulierungen verarbeitet, wobei die fertig applizierbaren Injektions- oder Infusionslösungen einen pH-Wert von etwa 2 - 7,4 und eine Pufferkapazität von bis zu 10 mVal/1 aufweisen.

2. Verfahren nach Anspruch 1, dadurch gekennzeichnet, daß die Injektions- oder Infusionslösungen eine Titrationsacidität von bis zu 10 mVal/1 aufweisen.

3. Verfahren nach Anspruch 1 oder 2, dadurch gekennzeichnet, daß als Humanproteine Erythropoietin oder G-CSF verwendet wird.

4. Verfahren nach Anspruch 1-3, dadurch gekennzeichnet, daß die Lösungen einen pH-Wert von etwa 3,8 - 7,2 aufweisen.

5. Verfahren nach Anspruch 4, dadurch gekennzeichnet, daß die Lösungen einen pH-Wert von etwa 4,5 - 7,2 aufweisen.

6. Verfahren nach Anspruch 1-5, dadurch gekennzeichnet, daß die Pufferkapazität bis zu 1 mmol/l beträgt.

7. Verfahren nach Anspruch 4, dadurch gekennzeichnet, daß die Pufferkapazität der Infusionslösung bis zu 0,3 mmol/l beträgt.

8. Verfahren nach Anspruch 5, dadurch gekennzeichnet, daß die Pufferkapazität der Injektionslösung bis zu 0,2 mmol/l beträgt.

9. Verfahren nach Anspruch 1-8, dadurch gekennzeichnet, daß die Titrationsacidität bis zu 5 mmol/l beträgt.

10. Verfahren nach Anspruch 1-8, dadurch gekennzeichnet, daß die Titrationsacidität bis zu 1 mmol/l beträgt.

11. Verfahren nach einem der Ansprüche 1-10, dadurch gekennzeichnet, daß die Arzneimittel eine gut verträgliche Puffersubstanz aus der Gruppe Glykokoll, Natriumcitrat, Kaliumphosphat, Natriumphosphat, Alkalicarbonate, Salze von Aminosäuren, Natrium- und Kaliumsalze der Apfelsäure, Maleinsäure, Fumarsäure, Weinsäure und Asparaginsäure oder Kombinationen dieser Substanzen enthalten.

12. Verfahren nach Anspruch 11, dadurch gekennzeichnet, daß die Puffersubstanz in der Infusions- oder Injektionslösung in einer Konzentration von 0,01 - 100 mmol/l enthalten ist.

13. Verfahren nach einem der Ansprüche 1 bis 12, dadurch gekennzeichnet, daß bei der Herstellung der Arzneimittel mindestens ein Konservierungsmittel zugesetzt wird.

14. Verwendung der nach dem Verfahren gemäß Ansprüche 1 - 13 hergestellten Humanprotein-haltigen pharmazeutischen Lösungen mit einem pH-Wert von 2 - 7,4 und einer Pufferkapazität von bis zu 10 mVal/1 zur Herstellung von gut verträglichen Arzneimitteln zur Anwendung als Injektions- oder Infusionslösung.

15. Pharmazeutische Verpackungseinheit umfassend einen ersten und einen zweiten Bestandteil, wobei der erste Bestandteil eine pharmazeutisch übliche Darreichungsform zur Herstellung von Humanprotein-haltigen Infusions- oder Injektionslösungen mit einem pH-Wert von etwa 2 - 7,4 und einer Pufferkapazität von bis zu 10 mVal/1 ist und der zweite Bestandteil aus einem Hinweis zur Applikation dieser Lösungen in gut verträglicher Form besteht.

16. Gut verträgliche Humanprotein-haltige Arzneimittel zur Anwendung als Injektions- oder Infusionslösung, dadurch gekennzeichnet, daß die fertig applizierbare Lösung einen pH-Wert von etwa 2 - 7,4 und eine Pufferkapazität von bis zu 10 mVal/1 aufweist.

17. Arzneimittel gemäß Anspruch 16, dadurch gekennzeichnet, daß die Titrationsacidität bis zu 10 mVal/1 beträgt.
